# EUROPEAN PATENT APPLICATION

(11) **EP 4 184 163 A1**
(43) Date of publication of application: **24.05.2023**
(21) Application number: 21843153.4
(22) Date of filing: 15.07.2021
(51) Int. Cl.: G01N 31/00, G01N 30/54, G01N 30/74

(54) **INSPECTION DEVICE**

(30) Priority: 17.07.2020 JP 2020122725
(71) Applicant: SHIMADZU CORPORATION, Kyoto-shi, Kyoto 604-8511 (JP)
(72) Inventor: IMAI, Akio, Tsukuba-shi, Ibaraki 305-8506 (JP); SHIMOTORI, Koichi, Tsukuba-shi, Ibaraki 305-8506 (JP); NAKAMORI, Akioki, Kyoto-shi, Kyoto 604-8511 (JP); NOTO, Kiyuki, Kyoto-shi, Kyoto 604-8511 (JP)
(74) Representative: Müller-Boré & Partner Patentanwälte PartG mbB
(86) International application number: PCT/JP2021/026573
(87) International publication number: WO 2022/014665

(57) **Abstract**

An object is to increase an efficiency of removing carbon dioxide derived from inorganic carbon. An inspection apparatus includes: a column that separates a group of substances contained in an aqueous sample by size; an addition unit that adds a reagent, which acidifies the aqueous sample, into a channel; a degassing unit provided downstream of the addition unit for degassing carbon dioxide; a measurement unit that measures organic carbon contained in each substance from which the carbon dioxide has been removed; and a temperature maintaining unit that maintains a temperature of each of the column and a container in which a gas-permeable tube of the degassing unit is housed.

## Description

### TECHNICAL FIELD

The present invention relates to an inspection apparatus that inspects an aqueous sample.

### BACKGROUND ART

In order to inspect an aqueous sample, an amount of a DOM (a dissolved organic matter) in water is inspected. In order to determine the amount of the DOM, an amount of TOC (total organic carbon) is used as an indicator for the inspection. The amount of TOC is determined by oxidizing an organic matter (organic carbon) to generate carbon dioxide, and measuring the generated carbon dioxide with an NDIR (nondispersive infrared) sensor or the like.

As methods of oxidizing organic carbon are known a method using a catalyst to combust it, a method using ultraviolet light, a two-step oxidation method, etc. (PTL 1).

Researches in recent years have revealed that decomposability varies depending on the DOM's molecular size. NPL 1 discloses an inspection apparatus comprising SEC (size-exclusion chromatography) and a TOC detector combined together to determine a molecular weight distribution of a DOM.

### CITATION LIST

### PATENT LITERATURE

PTL 1: Japanese Patent Laying-Open No. 2019-178902

### NON PATENT LITERATURE

NPL 1: Nobuyuki KAWASAKI, Kazuo MATSUSHIGE, Akio IMAI, Kazuhiro KOMATSU, Fumikazu OGISHI, Masato YAHATA, Hirohisa MIKAMI, and Takeshi GOTO, "Consideration for molecular weight distribution of DOC in Kasumigaura using size-exclusion chromatography equipped with TOC detector," The Japanese Society of Limnology, the 72nd Meeting, a collection of abstracts of lectures, Session ID: 3C5, September 2007

### SUMMARY OF INVENTION

### TECHNICAL PROBLEM

The inspection apparatus disclosed in NPL 1 that combines a SEC and a TOC detector can also detect organic carbon, which cannot be detected with an ultraviolet-visible spectrophotometer, a fluorometer, or the like.

The inspection apparatus disclosed in NPL 1 removes inorganic carbon by conversion into carbon dioxide, then oxidizes organic carbon, and detects carbon dioxide derived from organic carbon, thereby measuring a TOC amount. In this case, if carbon dioxide derived from inorganic carbon remains, it may cause a measurement error. It is thus required to increase an efficiency of removing carbon dioxide derived from inorganic carbon.

The present invention has been made to solve the above problem. An object of the present invention is to increase an efficiency of removing carbon dioxide derived from inorganic carbon.

### SOLUTION TO PROBLEM

An inspection apparatus according to an aspect of the present invention is an inspection apparatus for inspecting the water quality of an aqueous sample. The inspection apparatus includes: a column that separates a group of substances contained in the aqueous sample by size; an addition unit that adds a reagent into a channel to convert inorganic carbon included in each substance into carbon dioxide, the channel being connected to an outlet of the column, the reagent acidifying the aqueous sample flowing through the channel; a degassing unit provided downstream of the addition unit for degassing the carbon dioxide; and a measurement unit that oxidizes each substance from which the carbon dioxide has been removed by the degassing unit and measures organic carbon contained in each substance. The degassing unit includes a container, a gas-permeable tube disposed in the container and connected to the channel, and a pressure regulation unit that renders a pressure in the container negative with respect to the gas-permeable tube. The inspection apparatus further includes a temperature maintaining unit for maintaining a temperature of each of the column and the container.

### ADVANTAGEOUS EFFECTS OF INVENTION

The temperature maintaining unit, which is shared as the temperature maintaining unit that maintains the temperature of the column, maintains the temperature of the container with the gas-permeable tube therein, and accordingly, the solubility of carbon dioxide can be reduced, thereby increasing the efficiency of degassing carbon dioxide. Further, the temperature maintaining unit for maintaining the temperature of the column can be shared as the temperature maintaining unit for maintaining the temperature of the container with the gas-permeable tube therein, leading to a simplified configuration of the inspection apparatus.

### BRIEF DESCRIPTION OF DRAWINGS

Fig. 1 is a diagram schematically showing a configuration of an inspection apparatus 1 according to an embodiment.
Fig. 2 is a diagram schematically showing a configuration of a separation device 100 and a pretreatment unit 220.
Fig. 3 is a diagram schematically showing a configuration of an oxidation unit 242.
Fig. 4 is a diagram schematically showing a configuration of an inspection apparatus 1a according to a modified example.

### DESCRIPTION OF EMBODIMENTS

Hereinafter, an embodiment of the present invention will be described in detail with reference to the accompanying drawings. In the figures, identical or corresponding components are identically denoted and will not be described repeatedly.

### [Configuration of Inspection Apparatus 1]

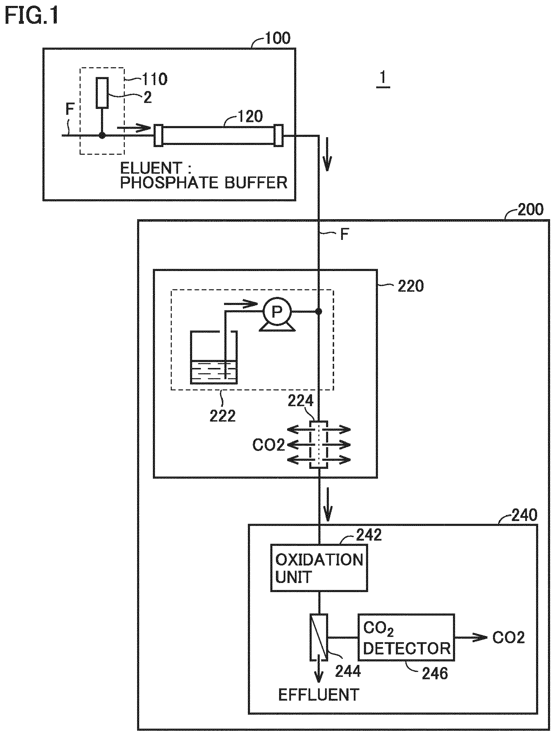

Fig. 1 is a diagram schematically showing a configuration of an inspection apparatus 1 according to an embodiment. Inspection apparatus 1 is an apparatus that inspects an aqueous sample 2. Referring to Fig. 1, inspection apparatus 1 comprises a separation device 100 and a TOC device 200 (or a measurement unit). Inspection apparatus 1 separates a substance in aqueous sample 2 by separation device 100 by size, oxidizes each substance eluted from separation device 100 in an order depending on size, and measures by TOC device 200 an amount of organic carbon (or an amount of TOC) contained in each substance. Thus, inspection apparatus 1 measures an amount of TOC for each organic matter dissolved in aqueous sample 2 having a different size.

Aqueous sample 2 inspected with inspection apparatus 1 is not limited as long as it is a sample mainly containing water. Examples of aqueous sample 2 may include tap water, well water, river or lake water, desalinated seawater, as well as artificial beverages and reagents. Inspection apparatus 1 is available not only for water quality inspection but also for inspection aimed at measuring organic carbon contained in a solution.

Separation device 100 separates by size a group of substances contained in aqueous sample 2 to be measured. Separation device 100 typically employs SEC to separate by size the group of substances contained in aqueous sample 2. Separation device 100 includes a sample injection unit 110 and a column 120.

Sample injection unit 110 injects aqueous sample 2 into a channel F that passes an eluent therethrough. Typically, a phosphate buffer can be used as the eluent. The eluent used is selected in view of the type of column 120 and an impact on TOC device 200 and TOC measurement.

Substances in aqueous sample 2 injected into channel F from sample injection unit 110 are separated by molecular size as the substances pass through column 120. More specifically, substances having larger molecular sizes (typically having larger molecular weights) are sequentially eluted from column 120 and sent to TOC device 200.

TOC device 200 measures an amount of TOC contained in an eluate (i.e., a mixture of a separated substance and the eluent) received from column 120. TOC device 200 includes a pretreatment unit 220 that removes inorganic carbon contained in the separated substance, and a measurement unit 240 that measures a total amount of carbon (i.e., an amount of TOC) contained in the substance after inorganic carbon is removed.

Pretreatment unit 220 receives an eluate from column 120 and acidifies the received eluate to convert inorganic carbon in aqueous sample 2 (in each separated substance) into carbon dioxide and thus remove it. Pretreatment unit 220 includes an addition unit 222 that adds into a channel a reagent that acidifies the eluate, and a degassing unit 224 that degasses carbon dioxide.

Addition unit 222 is provided at an inlet of TOC device 200. The reagent added by addition unit 222 is, for example, phosphoric acid, sulfuric acid, or the like. Addition unit 222 delivers the reagent to channel F by a pump P.

Degassing unit 224 is provided downstream of addition unit 222. Degassing unit 224 is typically a degasser, and degasses carbon dioxide derived from inorganic carbon generated by adding the reagent in addition unit 222. The eluate degassed in degassing unit 224 (a mixture of aqueous sample 2 (each separated substance) and the eluent) is delivered to measurement unit 240.

Measurement unit 240 oxidizes organic carbon in aqueous sample 2 (in each separated substance) having had inorganic carbon removed therefrom into carbon dioxide, and measures the generated carbon dioxide to measure an amount of TOC.

Measurement unit 240 includes an oxidation unit 242 that oxidizes organic carbon in aqueous sample 2 (in each separated substance) having had inorganic carbon removed therefrom, a gas-liquid separation unit 244 that separates carbon dioxide (or gas) generated by oxidizing organic carbon from a liquid, and a CO₂ detector 246 that measures the separated and thus obtained carbon dioxide.

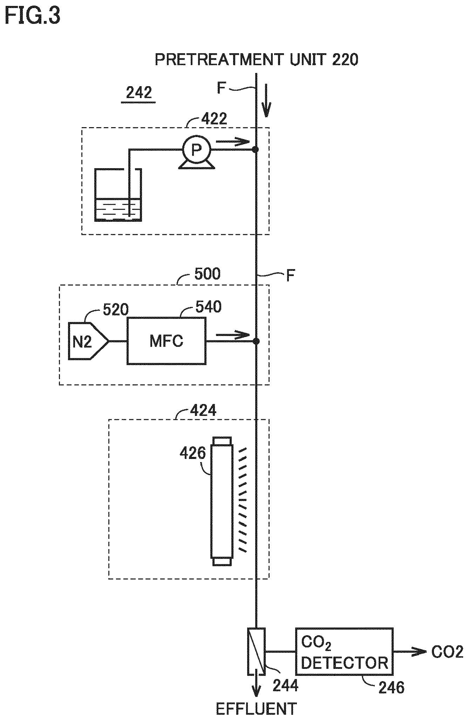

Oxidation unit 242 oxidizes organic carbon by a wet UV oxidation method. More specifically, oxidation unit 242 adds an oxidizing agent to each substance (eluate) having had inorganic carbon removed therefrom (or an eluate) and subsequently exposes it to ultraviolet light to oxidize organic carbon. A configuration of oxidation unit 242 will be described later with reference to Fig. 3. When the amount of TOC is lower than a predetermined amount, oxidation unit 242 may not add the oxidizing agent.

Gas-liquid separation unit 244 separates liquid from gas, and externally discharges the liquid as effluent and delivers the gas to CO₂ detector 246. The gas separated by gas-liquid separation unit 244 at least includes carbon dioxide generated by oxidizing organic carbon.

CO₂ detector 246 measures the concentration of the carbon dioxide in the gas delivered from gas-liquid separation unit 244. CO₂ detector 246 is typically a nondispersive infrared gas detector (a NDIR detector). CO₂ detector 246 is not limited to the NDIR detector, and may be any other detector that can measure carbon dioxide concentration.

### [Configuration of separation device 100 and pretreatment unit 220]

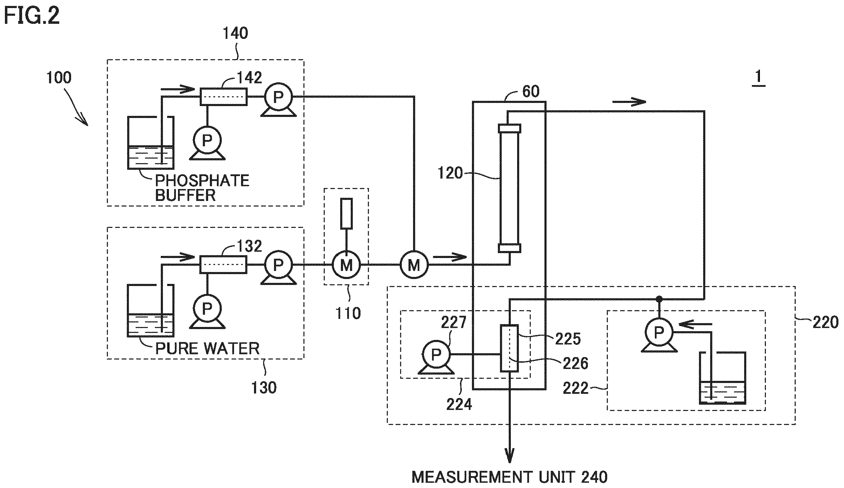

Fig. 2 is a diagram schematically showing a configuration of separation device 100 and pretreatment unit 220. In the present embodiment, an eluent is produced by delivering pure water and a phosphate buffer solution by separate pumps and mixing them in channel F.

Separation device 100 includes a first solvent delivery unit 130 that delivers water and a second solvent delivery unit 140 that delivers a phosphate buffer (a phosphate eluent). Herein, sample injection unit 110 is provided downstream of first solvent delivery unit 130. Second solvent delivery unit 140 is connected via a mixer M to some midpoint of a channel from sample injection unit 110 to column 120.

First solvent delivery unit 130 and second solvent delivery unit 140 include a first degassing unit 132 and a second degassing unit 142, respectively, as a pretreatment unit that degasses gas dissolved in a solvent.

Degassing unit 224 for removing carbon dioxide derived from inorganic carbon includes a container 225, a tube 226 disposed in container 225, and a vacuum pump 227 serving as a pressure regulation unit that renders the pressure in container 225 negative with respect to tube 226.

Tube 226 is connected to channel F passing aqueous sample 2 therethrough. Tube 226 is a gas permeable tube, and is made of a material allowing gas to permeate therethrough while preventing liquid from permeating therethrough. Tube 226 is typically, but not limited to, a gas permeable tube made of amorphous Teflon^{®} resin material, a hollow fiber membrane made of polytetrafluoroethylene, or the like.

When vacuum pump 227 reduces the pressure in container 225, the pressure in container 225 is rendered negative with respect to tube 226, and the gas in channel F moves to the outside of tube 226, thus removing, from an eluate (a mixture of each substance and an eluent), carbon dioxide derived from inorganic carbon. It suffices that the pressure in container 225 can be rendered negative with respect to tube 226, and the method used is not limited to a method involving the use of vacuum pump 227.

Herein, the solubility of carbon dioxide in an eluent can be reduced if container 225 with tube 226 therein can be maintained at high temperature, thus increasing the efficiency of degassing carbon dioxide. It is thus preferable to provide a temperature maintaining unit that maintains the temperature of container 225.

Inspection apparatus 1 further includes a column oven 60. Column oven 60 has a function of adjusting the temperature of column 120. Container 225 of degassing unit 224 for removing carbon dioxide derived from inorganic carbon is housed in column oven 60. In other words, column 120 and container 225 are housed in column oven 60. Thus, the temperatures of column 120 and container 225 are adjusted and maintained by column oven 60.

In other words, column oven 60 not only adjusts the temperature of column 120 but also functions as a temperature maintaining unit that maintains the temperature of container 225. The present embodiment illustrates an example in which column oven 60 for adjusting the temperature of column 120 functions as the temperature maintaining unit that maintains the temperature of container 225. In an alternative configuration, the temperature maintaining unit that maintains the temperature of container 225 may further function as a temperature maintaining unit for maintaining the temperature of column 120.

Although Fig. 2 illustrates an example in which the temperatures of column 120 and container 225 are adjusted by column oven 60, any other device may be used that maintains the temperatures of column 120 and container 225.

The set temperature of column oven 60 is preferably set to a temperature higher than a temperature outside column oven 60 (room temperature). The set temperature of column oven 60 is set to a recommended operating temperature of column 120.
For example, the set temperature of column oven 60 is not lower than the room temperature and not higher than 30 °C. The time for retention of each substance in column 120 tends to be shorter if the set temperature of column oven 60 is set higher, and accordingly, an analysis time can be reduced.

Usually, the entire column 120 is not at a constant temperature, and a problem such as a broad peak of a measurement result may occur if a temperature gradient occurs. Therefore, column oven 60 is usually used to maintain the temperature of column 120 at a constant temperature. In the present embodiment, the temperature in container 225 can be maintained to be not lower than a room temperature as container 225 is housed in column oven 60 that is usually used in use of column 120, and accordingly, the solubility of carbon dioxide in an eluent can be reduced, thereby improving the efficiency of degassing carbon dioxide. In the present embodiment, further, the temperature in container 225 can be maintained to be not lower than the room temperature by column oven 60 that is usually used in use of column 120, thus eliminating the need for newly preparing a device for maintaining the temperature of container 225 to be not lower than the room temperature.

### [Configuration of oxidation unit 242]

Fig. 3 is a diagram schematically showing a configuration of oxidation unit 242. Oxidation unit 242 includes an addition unit 422 and an irradiation unit 424. Addition unit 422 is provided at an inlet of oxidation unit 242. Irradiation unit 424 is provided downstream of addition unit 422.

Addition unit 422 adds an oxidizing agent to aqueous sample 2 having had inorganic carbon removed therefrom (or an eluate). The oxidizing agent is for example sodium persulfate. Addition unit 422 delivers the oxidizing agent to channel F by pump P.

Irradiation unit 424 includes a UV lamp 426 that irradiates aqueous sample 2 (or an eluate) passing through channel F with ultraviolet rays. Irradiation unit 424 includes a cylindrical UV lamp and a helical channel receiving ultraviolet rays from the UV lamp.

An inflow unit 500 is provided along channel F between addition unit 422 and irradiation unit 424. Inflow unit 500 causes gas to flow into channel F while controlling a flow rate of the gas. The flowing-in gas is gas containing no carbon compound that may be oxidized to generate carbon dioxide, and is, for example, nitrogen, helium, or oxygen. In the present embodiment, carbon-free gas is nitrogen.

Inflow unit 500 includes a nitrogen source 520 and a mass flow controller 540. Nitrogen gas is supplied from nitrogen source 520 to channel F. Mass flow controller 540 controls nitrogen gas supplied to channel F in flow rate.

More specifically, mass flow controller 540 controls nitrogen gas in flow rate so that the nitrogen gas is supplied from nitrogen source 520 to channel F at a fixed flow rate.

As bubbles of nitrogen gas are caused to flow through channel F together with the eluate, the bubbles of nitrogen gas exist in channel F. Since a portion including the bubbles of nitrogen gas has a less amount of eluate, the efficiency of irradiating the eluate with ultraviolet rays can be increased even when the same ultraviolet rays are applied.

After oxidation of organic carbon by irradiation unit 424, liquid and gas are separated from each other by gas-liquid separation unit 244. Then, only the gas is delivered to CO₂ detector 246, and the concentration of carbon dioxide generated through the oxidation of organic carbon is measured by CO₂ detector 246.

### [Inspection apparatus 1a according to a modified example]

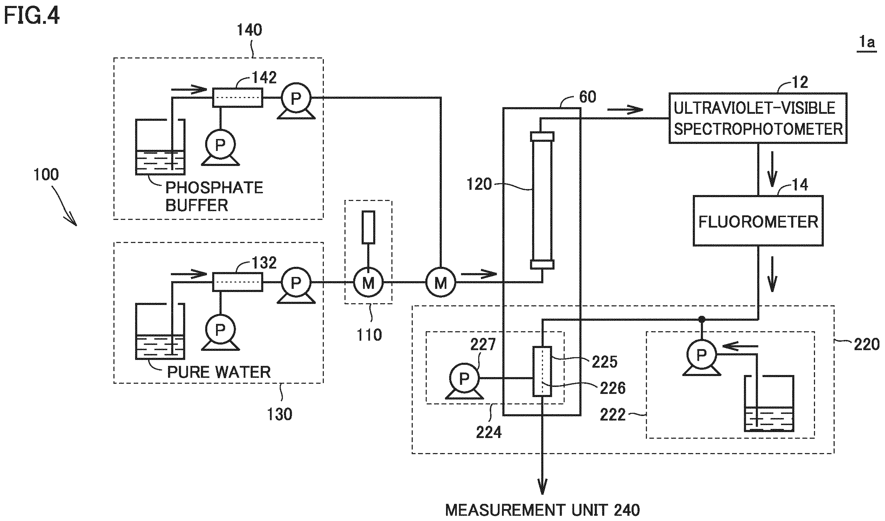

Fig. 4 is a diagram schematically showing a configuration of an inspection apparatus 1a according to a modified example. In the above embodiment, inspection apparatus 1 comprises TOC device 200 alone as a measurement device. Inspection apparatus 1 may comprise another measurement device in addition to TOC device 200. Inspection apparatus 1a according to the modified example differs from inspection apparatus 1 indicated in the above embodiment in that inspection apparatus 1a further comprises an ultraviolet-visible spectrophotometer 12 and a fluorometer 14 in addition to TOC device 200.

Ultraviolet-visible spectrophotometer 12 and fluorometer 14 are provided along channel F between separation device 100 and TOC device 200. More specifically, ultraviolet-visible spectrophotometer 12 and fluorometer 14 are provided on a route between column 120 and addition unit 222.

TOC device 200 subjects an aqueous sample to chemical treatment and thus measures carbon dioxide derived from organic carbon to measure an amount of TOC. In contrast, ultraviolet-visible spectrophotometer 12 and fluorometer 14 do not subject each separated substance to physical or chemical treatment and can thus measure the aqueous sample without changing a substance in composition, shape or function.

Accordingly, by disposing ultraviolet-visible spectrophotometer 12, fluorometer 14, and TOC device 200 in this order, each measurement device can be disposed on a single channel, and it is not necessary to branch an eluate that is eluted from separation device 100 into each measurement device. This can eliminate the necessity of reducing an amount of liquid of the aqueous sample used in each measurement device, and thus maintain measurement accuracy.

Inspection apparatus 1a according to the modified example comprises ultraviolet-visible spectrophotometer 12 and fluorometer 14. The inspection apparatus may comprise one of ultraviolet-visible spectrophotometer 12 and fluorometer 14 in addition to TOC device 200.

### [Aspects]

It is understood by those skilled in the art that the above-described exemplary embodiments are specific examples of the following aspects:
(Clause 1) An inspection apparatus according to an aspect is an inspection apparatus for inspecting an aqueous sample. The inspection apparatus includes: a column that separates a group of substances contained in the aqueous sample by size; an addition unit that adds a reagent into a channel to convert inorganic carbon included in each substance into carbon dioxide, the channel being connected to an outlet of the column, the reagent acidifying the aqueous sample flowing through the channel; a degassing unit provided downstream of the addition unit for degassing the carbon dioxide; and a measurement unit that oxidizes each substance from which the carbon dioxide has been removed by the degassing unit and measures organic carbon contained in each substance. The degassing unit includes a container, a gas-permeable tube disposed in the container and connected to the channel, and a pressure regulation unit (227) that renders a pressure in the container negative with respect to the gas-permeable tube. The inspection apparatus further includes a temperature maintaining unit for maintaining a temperature of each of the column and the container.
   With the inspection apparatus according to clause 1, the temperature of the container with the gas-permeable tube therein can be maintained by the temperature maintaining unit shared as the temperature maintaining unit that maintains the temperature of the column, and accordingly, the solubility of carbon dioxide can be reduced, thereby increasing the efficiency of degassing the carbon dioxide. Further, the temperature maintaining unit for maintaining the temperature of the column can be shared as the temperature maintaining unit for maintaining the temperature of the container with the gas-permeable tube therein, leading to a simplified configuration of the inspection apparatus.
(Clause 2) In the inspection apparatus according to clause 1, the temperature maintaining unit has a function of adjusting the temperature of each of the column and the container.
   The inspection apparatus according to clause 2 can make adjustment to the temperature that matches the measurement condition.
(Clause 3) The inspection apparatus according to clause 1 or 2 further includes a column oven that houses the temperature maintaining unit and the container.
   The inspection apparatus according to clause 3 does not need to include a temperature maintaining unit dedicated to the column and the degassing unit.
(Clause 4) The inspection apparatus according to any one of clauses 1 to 3 further includes a pretreatment unit that is provided upstream of an inlet of the column and degasses gas dissolved in the eluent.
   The inspection apparatus according to clause 4 can maintain the temperature of the container with the gas-permeable tube therein, thus increasing the efficiency of degassing carbon dioxide. Further, the temperature maintaining unit for maintaining the temperature of the column can be shared as the temperature maintaining unit for maintaining the temperature of the container with the gas-permeable tube therein, leading to a simplified configuration of the inspection apparatus.
(Clause 5) The inspection apparatus according to any one of clauses 1 to 4 further includes at least one of an ultraviolet-visible spectrophotometer and a fluorometer in a path from the column to the addition unit.

The inspection apparatus according to clause 5 can perform measurements in each measurement device without branching substances eluted from the column to the respective measurement devices. The substances eluted from the column do not need to be branched into the respective measurement devices, and accordingly, the amounts of the substances used in the respective measurement devices can be maintained, thus maintaining a measurement accuracy.

### REFERENCE SIGNS LIST

1, 1a inspection apparatus; 2 aqueous sample; 12 ultraviolet-visible spectrophotometer; 14 fluorometer; 60 column oven; 100 separation device; 110 sample injection unit; 120 column; 130 first solvent delivery unit; 132 first degassing unit; 140 second solvent delivery unit; 142 second degassing unit; 200 TOC device; 220 pretreatment unit; 222, 422 addition unit; 224 degassing unit; 225 container; 226 tube; 227 vacuum pump; 240 measurement unit; 242 oxidation unit; 244 gas-liquid separation unit; 246 CO₂ detector; 424 irradiation unit; 426 UV lamp; 500 inflow unit; 520 nitrogen source; 540 mass flow controller; F channel ; M mixer; P pump.

## Claims

1. An inspection apparatus for inspecting an aqueous sample, the inspection apparatus comprising:
a column that separates a group of substances contained in the aqueous sample by size;
an addition unit that adds a reagent into a channel to convert inorganic carbon included in each substance into carbon dioxide, the channel being connected to an outlet of the column, the reagent acidifying the aqueous sample flowing through the channel;
a degassing unit provided downstream of the addition unit for degassing the carbon dioxide; and
a measurement unit that oxidizes each substance from which the carbon dioxide has been removed by the degassing unit and measures organic carbon contained in each substance,
the degassing unit including
a container,
a gas-permeable tube disposed in the container and connected to the channel, and
a pressure regulation unit that renders a pressure in the container negative with respect to the gas-permeable tube,
the inspection apparatus further comprising a temperature maintaining unit for maintaining a temperature of each of the column and the container.

2. The inspection apparatus according to claim 1, wherein the temperature maintaining unit has a function of adjusting the temperature of each of the column and the container.

3. The inspection apparatus according to claim 1 or 2, further comprising a column oven that houses the temperature maintaining unit and the container.

4. The inspection apparatus according to claim 1, further comprising a pretreatment unit that is provided upstream of an inlet of the column and degasses gas dissolved in an eluent.

5. The inspection apparatus according to claim 1, further comprising at least one of an ultraviolet-visible spectrophotometer and a fluorometer in a path from the column to the addition unit.
